(19) Europäisches Patentamt
European Patent Office
Office européen des brevets

(11) Veröffentlichungsnummer: **0 048 376**
**B1**

# (12) EUROPÄISCHE PATENTSCHRIFT

(45) Veröffentlichungstag der Patentschrift: **15.02.84**

(51) Int. Cl.³: **C 07 C 125/067,** C 07 D 307/52, C 07 D 309/04

(21) Anmeldenummer: **81107017.6**

(22) Anmeldetag: **07.09.81**

**(54) Verfahren zur Herstellung von N-substituierten Imido-dicarbonsäure-diarylestern.**

(30) Priorität: **19.09.80 DE 3035392**

(43) Veröffentlichungstag der Anmeldung: **31.03.82 Patentblatt 82/13**

(45) Bekanntmachung des Hinweises auf die Patenterteilung: **15.02.84 Patentblatt 84/7**

(84) Benannte Vertragsstaaten: **BE CH DE FR GB IT LI NL**

(56) Entgegenhaltungen:
**GB - A - 484 683**

**HOUBEN-WEYL "Methoden der organischen Chemie", 1952, 4. Auflage, Band VIII: "Sauerstoffverbindungen III" GEORG THIEME VERLAG, Stuttgart**

(73) Patentinhaber: **BAYER AG, Zentralbereich Patente, Marken und Lizenzen, D-5090 Leverkusen 1, Bayerwerk (DE)**

(72) Erfinder: **Dickoré, Karlfried, Dr., Nicolai-Hartmann-Strasse 19, D-5090 Leverkusen (DE)**
Erfinder: **Kühle, Engelbert, Dr., von Bodelschwingh-Strasse 42, D-5060 Berg.-Gladbach 2 (DE)**

**EP 0 048 376 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Verfahren zur Herstellung von N-substituierten Imidodicarbonsäure-diarylestern

Die Erfindung betrifft ein neues einstufiges Verfahren zur Herstellung von N-substituierten Imido-dicarbon-säure-diarylestern, welche als Zwischenprodukte für die Synthese von Herbiziden verwendet werden können.

Schon seit längerem bekannt ist ein Verfahren zur Herstellung von N-substituierten Imido-dicarbonsäure-dialkylestern durch Umsetzung von N-substituierten Carbamidsäure-alkylestern mit Kohlensäure-alkylester-chloriden in Gegenwart von metallischem Natrium (vgl. J. Amer. Chem. Soc. 69, 2616–2618 [1947]). Wie weiter unten näher erläutert wird, ist es jedoch unerwarteterweise nicht möglich, dieses vorbekannte Verfahren auf die Herstellung entsprechender O,O-Diarylester zu übertragen.

Es ist weiterhin bekannt, dass man bestimmte N-Methyl-imido-dicarbonsäure-diarylester (welche insektizide und akarizide Eigenschaften besitzen) herstellen kann, indem man N-Chlorcarbonyl-N-methylcarbamidsäure-arylester mit Phenolen in Gegenwart eines Verdünnungsmittels und gegebenenfalls in Gegenwart eines Säurebindemittels bei Temperaturen zwischen 0 und 100°C, vorzugsweise zwischen 20 und 40°C, umsetzt (vgl. DE-A 2 132 936). Dieses Verfahren hat jedoch den technischen Nachteil, dass die für die Herstellung von homologen N-Alkylderivaten als Ausgangsstoffe benötigten N-Chlorcarbonyl-N-alkylcarbamid-säurearylester zum Teil nicht bekannt und in jedem Falle nur durch ausserordentlich aufwendige, mehrstufige Verfahren und mit mässigen Ausbeuten zugänglich sind. Dies gilt insbesondere für Ausgangsstoffe mit höheren, verzweigten N-Alkylgruppen, so dass das genannte Verfahren in der praktischen Anwendbarkeit im wesentlichen auf die Synthese von N-Methyl-derivaten beschränkt ist.

Nach einer eigenen vorgängigen Patentanmeldung (EP-A-0 034 750) ist es möglich, N-substituierte Imido-dicarbonsäure-diarylester herzustellen, indem man entsprechende Carbamidsäure-arylester mit Kohlensäure-arylester-halogeniden, gegebenenfalls in Gegenwart eines Verdünnungsmittels, bei erhöhten Temperaturen (100–300°C) umsetzt. Die benötigten Carbamidsäure-arylester müssen in einer besonderen Verfahrensstufe aus den entsprechenden primären Aminen durch Umsetzung mit Kohlensäure-arylester-halogeniden oder Kohlensäure-diarylestern hergestellt werden (vgl. z.B. Houben-Weyl: Methoden der organischen Chemie, 4. Auflage, Vol. VIII, Sauerstoffverbindungen III, Stuttgart (1952), Seite 138).

Es wurde nun überraschend gefunden, dass man die N-substituierten Imido-dicarbonsäure-diarylester der allgemeinen Formel I

$R^1$–N(CO–O$R^2$)$_2$ (I),

in welcher

$R^1$ für einen geradkettigen oder verzweigten Alkylrest mit 1–10 C-Atomen, der gegebenenfalls durch Niederalkoxy, Niederalkylmercapto, Halogen, Cyano oder Nitro substituiert sein kann; einen Alkylrest mit 3–8 C-Atomen; einen Alkinylrest mit 3–8 C-Atomen; einen cycloaliphatischen Rest mit 3–8 C-Atomen, der gegebenenfalls durch Niederalkyl substituiert sein kann; einen araliphatischen Rest mit 7–12 C-Atomen, wobei das aromatische Ringsystem gegebenenfalls durch Halogen, Nitro, Trifluormethyl, Cyano, Niederalkyl und/oder Niederalkoxy substituiert sein kann; einen aromatischen Rest mit 6–12 C-Atomen, der gegebenenfalls durch Halogen, Nitro, Trifluormethyl, Cyano, Niederalkyl und/oder Niederalkoxy substituiert sein kann, oder für einen heterocyclischen Rest mit 5–6 Ringatomen, wobei 1–3 Heteroatome im Ringsystem vorhanden sein können, steht, und

$R^2$ für einen gegebenenfalls durch Chlor, Methyl und/oder Methoxy substituierten Phenyl- oder Naphthylrest steht,

erhält, wenn man primäre Amine der allgemeinen Formel II

$R^1$–$NH_2$ (II),

in welcher $R^1$ die oben angegebene Bedeutung hat,

mit Kohlensäure-arylester-halogeniden der allgemeinen Formel III

X–CO–O$R^2$ (III),

in welcher

$R^2$ die oben angegebene Bedeutung hat und

X für Halogen steht,

in Gegenwart eines Verdünnungsmittels, ohne Zusatz eines Säurebindemittels, bei Temperaturen zwischen 100 und 300°C umsetzt.

Es ist als überraschend zu bezeichnen, dass die erfindungsgemässe Umsetzung, die sehr wahrscheinlich über die Zwischenstufe der entsprechenden Carbamidsäure-arylester ($R^1$–NH–CO–O$R^2$) verläuft, glatt und in hohen Ausbeuten zu den gewünschten Imido-dicarbonsäure-diarylestern (I) führt, da Carbamidsäure-arylester mit Kohlensäure-arylester-halogeniden (III) in Gegenwart eines säurebindenden Mittels nicht in gewünschter Weise reagieren:

Es ist zwar bekannt – wie oben bereits erwähnt –, dass man N-substituierte Imido-dialkylester durch Umsetzung von N-substituierten Carbamidsäure-alkylestern mit Kohlensäure-alkylester-chloriden in Gegenwart von metallischem Natrium herstellen kann (vgl. J. Amer. Chem. Soc. 69, 2616–2618 [1947]).

Die Übertragung dieser Methode auf die entsprechenden Carbamidsäure-arylester versagt jedoch völlig. Beispielsweise reagiert Neopentylcarbamidsäure-phenylester mit Kohlensäure-phenylester-chlorid unter den in der zuletzt genannten Veröffentlichung beschriebenen Reaktionsbedingungen ausschliesslich zu Neopentylisocyanat und Diphenylcarbonat.

Auch mit Butyl-lithium als Säurefänger wird kein Neopentylimido-dicarbonsäure-diphenylester gebildet. Umso überraschender ist es, dass bei erhöhter Temperatur und in Abwesenheit eines Säurefängers die erfindungsgemässe Reaktion glatt abläuft. Nach dem weiterhin bekannten Stand der Technik wäre zu erwarten gewesen, dass bei hoher Temperatur eine totale Rückspaltung der zunächst gebildeten Carbamidsäure-arylester in Isocyanat und Phenol erfolgen würde (vgl. Houben-Weyl: Methoden der organischen Chemie, 4. Aufl. Bd. 8, S. 127 [1952]).

Verwendet man Neopentylamin und Kohlensäure-phenyl-ester-chlorid als Ausgangsstoffe, so kann der Reaktionsverlauf durch das folgende Reaktionsschema wiedergegeben werden:

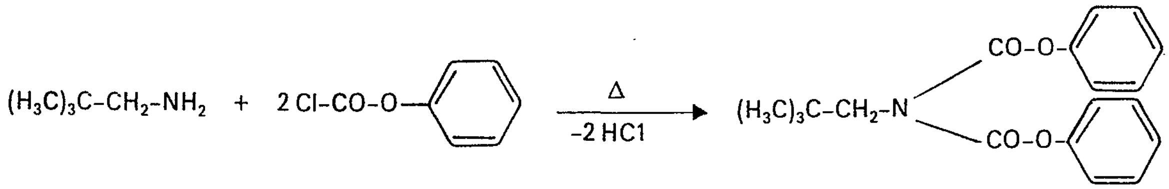


Die als Ausgangsstoffe zu verwendenden primären Amine sind durch die Formel (II) allgemein definiert. In dieser Formel steht $R^1$ für einen geradkettigen oder verzweigten Alkylrest mit 1-10 C-Atomen, der gegebenenfalls durch Niederalkoxy, Niederalkylmercapto, Halogen (insbesondere Fluor und Chlor), Cyano oder Nitro substituiert sein kann; einen Alkenylrest mit 3-8 C-Atomen; einen Alkinylrest mit 3-8 C-Atomen; einen cycloaliphatischen Ring mit 5-8 Ring-C-Atomen, der gegebenenfalls durch Niederalkyl substituiert sein kann; einen araliphatischen Rest mit insgesamt 7-12 C-Atomen, wobei das aromatische Ringsystem gegebenenfalls durch Halogen, Nitro, Trifluormethyl, Cyano, Niederalkyl und/oder Niederalkoxy substituiert sein kann; einen aromatischen Rest mit 6-12 C-Atomen, der gegebenenfalls durch Halogen, Nitro, Trifluormethyl, Cyano, Niederalkyl und/oder Niederalkoxy substituiert sein kann, oder für einen heterocyclischen Rest mit 5-6 Ringatomen, wobei 1-3 Heteroatome, wie Sauerstoff, Schwefel und/oder Stickstoff im Ringsystem vorhanden sein können. Die Ausdrücke «Niederalkyl», «Niederalkoxy» bzw. «Niederalkylmercapto» sollen im Rahmen dieser Erfindung entsprechende Reste mit jeweils 1-4 C-Atomen bedeuten.

Die erfindungsgemäss verwendbaren primären Amine der Formel (II) sind bereits bekannt oder können nach üblichen, bekannten Verfahren hergestellt werden (vgl. z.B. Houben-Weyl, Methoden der organischen Chemie, 4. Auf. Bd. XI/1, S. 9-1033 [1957]).

Als Beispiele für primäre Amine der Formel (II) seinen im einzelnen genannt:

Methylamin
Ethylamin
Propylamin
Isopropylamin
1,1-Dimethyl-propylamin
1,2-Dimethyl-propylamin
2,2-Dimethyl-propylamin (Neopentylamin)
1,2,2-Trimethyl-propylamin
1-Ethyl-propylamin
1-Isopropyl-2-methyl-propylamin
Butylamin
Isobutylamin
sek.-Butylamin
tert.-Butylamin
1-Methyl-butylamin
2,2-Dimethyl-butylamin
3,3-Dimethyl-butylamin
1,3,3-Trimethyl-butylamin
Pentylamin
1-Methyl-pentylamin
2,2-Dimethyl-pentylamin
1,2,2-Trimethyl-pentylamin
Hexylamin
2-Ethyl-hexylamin
1-Methyl-oktylamin
Allylamin
2-Methyl-allylamin
Propargylamin
Cyclopropylamin
Cyclopropyl-methylamin
Cyclobutylamin
Cyclohexen(3)-yl-methylamin
3,4-Dimethyl-cyclohexen(3)-yl-methylamin
Cycloheptanylamin
Cycloheptanyl-methylamin
Cyclooktanylamin
Cyclooktanyl-methylamin
Cyclododekanylamin
Cyclododekanyl-methylamin
Adamantyl-methylamin
2-(Bicyclo[2,2,1]heptyl)-methylamin
6-(2,3,3a,4,5,6,7,7a-Oktahydro-4,7-methano-indenyl-methylamin
2-(1,2,3,4,5,6,7,8,8a,4a-Dekahydro-1,4:5,8-dimethano-napthyl)-methylamin
5-(4,5,6,7,7a,3a-Hexahydro-indenyl)-methylamin
2-Chlor-ethylamin
2,2,2-Trifluor-ethylamin
3,3-Dichlor-3-fluor-propylamin
3,3,3-Trifluor-propylamin
2,2-Difluor-propylamin
2,2,2-Trifluor-1-methyl-ethylamin
6-Chlor-hexylamin
3-Trifluormethyl-cyclohexylamin
4-Trifluormethyl-cyclohexylamin
3-Trifluormethyl-cyclohexyl-methylamin
4-Trifluormethyl-cyclohexyl-methylamin

2-Methoxy-ethylamin
3-Methoxy-propylamin
5-Cyano-pentylamin
2-Ethoxycarbonyl-ethylamin
Anilin
2-Chloranilin
3-Chloranilin
4-Chloranilin
2,4-Dichloranilin
3,4-Dichloranilin
3,5-Dichloranilin
2,4,5-Trichloranilin
3-Nitroanilin
4-Nitroanilin
3-Chlor-4-nitroanilin
2-Methylanilin
2-Chlor-6-methylanilin
4-Chlor-2-trifluormethylanilin
3-Methylanilin
3-Trifluormethylanilin
4-Methylanilin
2,6-Dimethylanilin
2-Ethylanilin
2-Ethyl-6-methylanilin
2,6-Diethylanilin
Benzylamin
1-Phenyl-ethylamin
2-Phenyl-ethylamin
2-Chlor-benzylamin
2,4-Dichlor-benzylamin
Furyl(2)-methylamin
Tetrahydro-furyl(2)-methylamin
Tetrahydro-pyranyl(2)-methylamin
Tetrahydro-pyranyl(3)-methylamin.

Die weiterhin als Ausgangsstoffe zu verwendenden Kohlensäure-arylester-halogenide sind durch die Formel (III) allgemein definiert. In dieser Formel steht $R^2$ für einen gegebenenfalls durch Chlor, Methyl und/oder Methoxy substituierten Phenyl- oder Naphthylrest; X in Formel (III) steht vorzugsweise für Chlor oder Fluor.

Die erfindungsgemäss verwendbaren Kohlensäure-aryl-ester-halogenide der Formel (III) sind bekannt oder können nach bekannten Verfahren hergestellt werden. So kann man z.B. die Kohlensäure-phenylester-chloride in an sich bekannter Weise durch Phosgenierung von Phenolen herstellen (vgl. Houben-Weyl, Methoden der organischen Chemie, 4. Aufl., Bd. 8, S. 103 [1952]); die entsprechenden Kohlensäure-phenylesterfluoride können analog aus Phenolen und Difluorphosgen gewonnen werden (vgl. J. Chem. Soc. [London] 1948, S. 2183).

Als Beispiele für die Ausgangsverbindungen der Formel (III) seinen im einzelnen genannt: die Kohlensäureester-chloride des Phenols, 4-Chlorphenols, 4-Kresols oder 1-Naphthols sowie das Kohlensäureester-fluorid des Phenols.

Die erfindungsgemässe Umsetzung wird unter Verwendung eines Verdünnungsmittels für das primäre Amin (II) durchgeführt.

Zweckmässigerweise geht man so vor, dass das Kohlensäurearylester-halogenid (III) vorgelegt und auf die Reaktionstemperatur erwärmt wird und dass eine Lösung des primären Amins in einem geeigneten Verdünnungsmittel langsam zugeführt wird. Als Verdünnungsmittel für das zuzugebende Amin sind niedrig siedende inerte organische Lösungsmittel, wie Kohlenwasserstoffe und chlorierte Kohlenwasserstoffe, z.B. Petrolether, Cyclohexan, Dichlormethan, Chloroform oder Difluordichlormethan, vorzugsweise Petrolether (Siedebereich 30–50°C) geeignet; diese Lösungsmittel verdampfen bei der Reaktionstemperatur und dienen dadurch gleichzeitig als «Schleppmittel» zur Abführung des gebildeten Halogenwasserstoffs.

Ausserdem ist es möglich und in vielen Fällen besonders vorteilhaft, die Reaktion in einem Überschuss des als Reaktionskomponente dienenden Kohlensäure-aryl-ester-halogenids (III) durchzuführen.

Auch das Kohlensäure-arylester-halogenid (III), das im allgemeinen vorgelegt wird, kann in Form einer verdünnten Lösung eingesetzt werden. Als hierfür geeignete Verdünnungsmittel kommen hochsiedende inerte organische Lösungsmittel, wie chlorierte oder nitrierte aromatische Kohlenwasserstoffe, z.B. Chlorbenzol, die Dichlorbenzole, die Trichlorbenzole oder Nitrobenzol, in Frage. Jedoch bringt die Verwendung eines solchen zusätzlichen Verdünnungsmittels im allgemeinen keinen nennenswerten Vorteil. Es hat sich als zweckmässiger erwiesen, ohne ein solches zusätzliches Verdünnungsmittel zu arbeiten und stattdessen das Kohlensäure-arylester-halogenid (III) in überschüssigen Mengen einzusetzen.

Führt man die erfindungsgemässe Umsetzung ohne Verwendung eines zusätzlichen Verdünnungsmittels für das (vorgelegte) Kohlensäure-arylester-halogenid (III) durch, so kann man auf 1 Mol eines primären Amins der Formel (II) bis zu 20 Mol, zweckmässigerweise jedoch etwa 4–15 Mol, bevorzugt etwa 8–12 Mol Kohlensäure-arylester-halogenid einsetzen.

Verwendet man ein Verdünnungsmittel auch für das Kohlensäure-arylester-halogenid (III), so setzt man auf 1 Mol des primären Amins (II) im allgemeinen 2–15, vorzugsweise 3–12 Mol Kohlensäure-aryl-ester-halogenid (III) ein. Es empfiehlt sich also auch im letztgenannten Falle, das Kohlensäure-aryl-ester-halogenid (III) in überstöchiometrischen Mengen einzusetzen.

Das erfindungsgemässe Verfahren wird ohne Zusatz eines Säurebindemittels durchgeführt. Durch Verwendung eines niedrig siedenden, inerten organischen Lösungsmittels als Verdünnungsmittel für das primäre Amin gelingt es jedoch, einen grossen Teil des im Verlaufe der Reaktion gebildeten Halogenwasserstoffes aus dem Reaktionsgemisch zu entfernen, da das Lösungsmittel wie oben erwähnt zugleich als «Schleppmittel» dient. Um die Austreibung des gebildeten Halogenwasserstoffes zu vervollständigen, ist es ratsam, zusätzlich einen ständigen Luft- oder Stickstoffstrom durch das Reaktionsgemisch zu leiten, (vgl. Herstellungsbeispiele).

Die Reaktionstemperaturen können in einem grösseren Bereich variiert werden. Im allgemeinen arbeitet man, wie oben angegeben, zwischen etwa 100 und 300°C, vorzugsweise zwischen etwa 170 und 250°C.

Im allgemeinen wird die erfindunsgemässe Reaktion bei Normaldruck ausgeführt.

Die Isolierung der Reaktionsprodukte erfolgt in einfacher Weise durch destillative Trennung des Reaktionsgemisches. Feste, höher schmelzende Imido-dicarbonsäure-diarylester lassen sich auch leicht durch Umkristallisieren reinigen.

Die erfindungsgemässen neuen N-substituierten Imido-dicarbonsäure-diarylester (I) können als Zwischenprodukte zur Herstellung bekannter herbizider Wirkstoffe aus der Reihe der 1,3,5-Triazin-2,4(1H,3H)-dione verwendet werden (vgl. z.B. DE-OS 2 254 200 und US-PS 4 056 527).

Nach einem Verfahren, welches Gegenstand einer vorgängigen Patentanmeldung ist (vgl. EP-A-0 034 751), kann man 1,3,5-Triazin-2,4-(1H, 3H)-dione der allgemeinen Formel

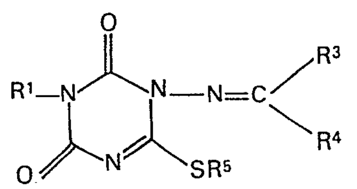

(IV)

in welcher
$R^1$ die oben angegebene Bedeutung hat und
$R^3$, $R^4$ und $R^5$ jeweils für gleiche oder verschiedene Alkylreste stehen,
mit hoher Ausbeute und Reinheit herstellen, wenn man die erfindungsgemässen N-substituierten Imido-dicarbonsäure-diarylester der allgemeinen Formel

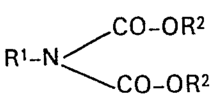

(I)

in welcher
$R^1$ und $R^2$ die oben angegebene Bedeutung haben,
mit einem Isothiosemicarbazon der allgemeinen Formel

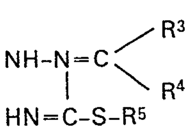

(V),

in welcher
$R^3$, $R^4$ und $R^5$ die oben angegebene Bedeutung haben,
in etwa stöchiometrischen Mengen, ohne Verwendung eines Verdünnungsmittels und ohne Zusatz einer Base als Hilfsstoff, bei Temperaturen zwischen 50 und 150°C, vorzugsweise zwischen 70 und 120°C, umsetzt.

Die Aufarbeitung und Isolierung der Triazindione (IV) kann z.B. in der Weise erfolgen, dass man das bei der Kondensationsreaktion, (I) + (V) → (IV), gebildete (gegebenenfalls substituierte) Phenol im Vakuum abdestilliert und den Rückstand durch Destillation im Hochvakuum oder durch Umlösen, falls erforderlich, reinigt.

Die so hergestellten 1,3,5-Triazin-2,4(1H,3H)-dione (IV) sind selbst herbizide Wirkstoffe; sie können jedoch durch Hydrolytische Abspaltung des als Schutzgruppe dienenden Alkylidenrestes ($=CR^3R^4$) auch leicht in die entsprechenden 1-Amino-1,3,5-triazin-2,4(1H,3H)-dione der allgemeinen Formel

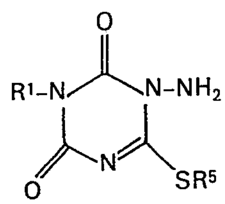

(VI),

in welcher
$R^1$ und $R^5$ die oben angegebene Bedeutung haben,
die ebenfalls ausgezeichnete Herbizide sind, umgewandelt werden. Ferner können die S-Alkylreste ($-SR^5$) in (IV) und (VI) durch Umsetzung mit primären oder sekundären Aminen gegen Alkylamino- bzw. Dialkylaminogruppen ausgetauscht werden, wobei ebenfalls bekannte herbizide Wirkstoffe erhalten werden (vgl. ebenfalls DE-OS 2 254 200 und US-PS 4 056 527).

Das hier angegebene neue, von den erfindungsgemässen Imido-dicarbonsäure-diarylestern (I) ausgehende Herstellungsverfahren für die herbiziden Wirkstoffe der allgemeinen Formeln (IV) und (VI) sowie deren 6-Amino-derivate hat gegenüber den, z.B. aus DE-OS 2 254 200, vorbekannten Verfahren erhebliche und überraschende Vorteile. So kann man die Cyclisierungsreaktion ohne Verwendung von Lösungsmitteln in der Schmelze der Ausgangsprodukte durchführen. Hierbei werden keine weiteren Hilfsstoffe, wie z.B. organische Basen, benötigt. Einziges Nebenprodukt sind Phenole (keine Halogenwasserstoffe!), welche leicht abgetrennt und weiterverwendet werden können. Schliesslich sind die als Ausgangsstoffe eingesetzten Imido-dicarbonsäure-diarylester (I) nach dem hier beanspruchten Verfahren auf technisch einfache Weise aus leicht zugänglichen Vorprodukten in hohen Ausbeuten herstellbar.

Die Isothiosemicarbazone der allgemeinen Formel (V) sind bekannt oder können nach bekannten Verfahren hergestellt werden, z.B. durch S-Alkylierung von Thiosemicarbazonen (vgl. Houben-Weyl, Methoden der organischen Chemie, 4. Auflage, Band 9, S. 912).

Nachfolgend wird beispielhaft die Synthese des besonders wirksamen herbiziden Wirkstoffes

1-Amino-6-ethylthio-3-neopentyl-1,3,5-triazin-2,4-
(1H,3H)-dion (VIa) vgl. z.B. DK-PS 136 067), aus-

gehend von der erfindungsgemässen Verbindung N-Neopentyl-imido-dicarbonsäure-diphenylester (Ia) beschrieben; der Reaktionsverlauf kann durch das folgende Formelschema wiedergegeben werden:

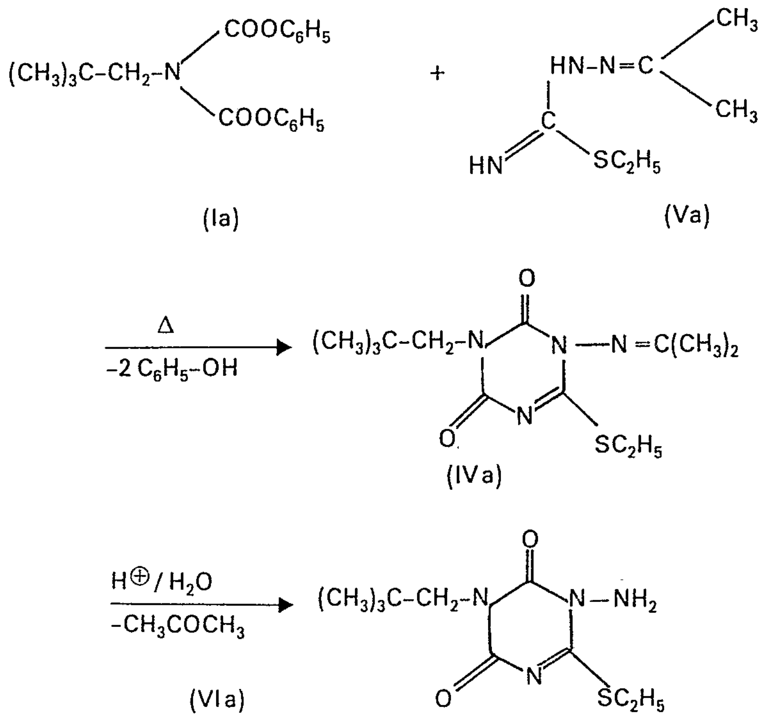


(Ia) (Va) (IVa) (VIa)

Eine Mischung von 65,4 (0,2 Mol) N-Neopentyl-imido-dicarbonsäure-diphenylester (vgl. Herstellungsbeispiel 1) und 31,8 g (0,2 Mol) Aceton-S-ethyl-isothiosemicarbazon (Va) wird aufgeschmolzen und 5 Stunden bei 100°C gerührt. Danach wird das gebildete Phenol im Vakuum abdestilliert. Der Rückstand, der im wesentlichen aus 1-Isopropylidenamino-6-ethyl-thio-3-neopentyl-1,3,5-triazin-2,4 (1H,3H)-dion (IVa) besteht, wird in 200 ml Isopropanol gelöst. Zur hydrolytischen Abspaltung der Isopropyliden-Schutzgruppe gibt man 2,8 g p-Toluolsulfonsäure zu und tropft bei einer Temperatur von 60°C und einem Druck von 200–300 mbar innerhalb einer halben Stunde 14,4 ml Wasser zu. Das gebildete Aceton wird während der Reaktion zusammen mit etwa 100 ml Isopropanol abdestilliert. Das auskristallisierte 1-Amino-6-ethylthio-3-neopentyl-1,3,5-triazin-2,4-(1H,3H)-dion (VIa) wird bei 0°C abgesaugt und mit Methanol gewaschen. Man erhält 38,2 g (VIa) vom Schmelzpunkt 202°C, entsprechend einer Ausbeute von 74% der Theorie.

In analoger Weise kann, ausgehend von der erfindungsgemässen Verbindung N-Isobutyl-imido-dicarbonsäure-diphenylester (Ib), das bekannte, herbizid wirksame 1-Amino-3-isobutyl-6-methylthio-1,3,5-triazin-2,4(1H,3H)-dion (VIb) (vgl. z.B. DK-PS 13 60 67) hergestellt werden, wobei das Zwischenprodukt 1-Isopropylidenamino-3-isobutyl-6-methylthio-1,3,5-triazin-2,4(1H,3H)-dion (IVb) isoliert werden kann:

1. Stufe

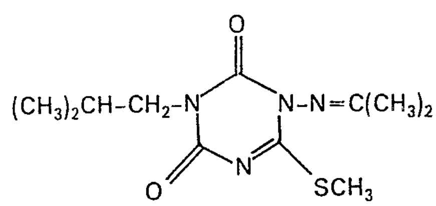

(IVb)

34,6 g (0,11 Mol) N-Isobutyl-imido-dicarbonsäure-diphenylester (Ib) (vgl. Herstellungsbeispiel 5) und 16,0 g (0,11 Mol) Aceton-S-methylisothiosemicarbazon werden bei 50°C aufgeschmolzen und in einem Ölbad von 100°C 4 Stunden lang gerührt. Man destilliert das gebildete Phenol bei einem Druck von 18 mbar ab, wobei man die Bad-Temperatur bis auf 140°C steigert. Der Rückstand (30,3 g) erstarrt; er wird mit 150 ml Cyclohexan aufgekocht, wobei 22,4 g reines 1-Isopropylidenamino-3-isobutyl-6-methylthio-1,3,5-triazin-2,4(1H,3H)-dion (IVb) vom Schmelzpunkt 125–127°C ungelöst zurückbleiben. Aus dem Filtrat davon kristallisieren weitere 6,4 g (IVb). Die Gesamtausbeute beträgt 28,8 g (97% d.Th.). Die Verbindung (IVb) kann destilliert werden: Siedepunkt 165°C bei 0,38 mbar.

2. Stufe

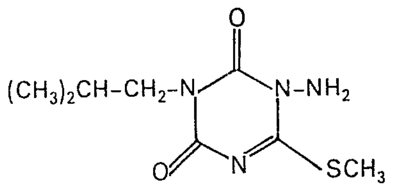

(IV b)

In einer Destillationsapparatur löst man 27,0 g (0,1 Mol) der Verbindung (IVb) bei 60°C in 200 ml Isopropanol und stellt einen Druck von 260–200 mbar ein, so dass das Lösungsmittel zu sieden beginnt und im absteigenden Kühler kondensiert. Die Innentemperatur beträgt 45–50°C. Sodann tropft man innerhalb 15 Minuten eine Lösung von 0,4 ml konzentrierter Schwefelsäure in 7 ml Wasser zu, wobei man während dieser Zeit ca. 70 ml Isopropanol, zusammen mit dem gebildeten Aceton, abdestilliert. Aus der verbleibenden Lösung kristallisieren bei 0°C 14,5 g 1-Amino-3-isobutyl-6-methylthio-1,3,5-triazin-2,4(1H,3H)-dion (VIb) vom Schmelzpunkt 167–169°C aus; aus dem eingeengten Filtrat davon werden weitere 4,5 g erhalten. Die Gesamtausbeute von 19,0 g entspricht 83% der Theorie.

Die nachfolgenden Herstellungsbeispiele sollen zur weiteren Erläuterung der Erfindung dienen.

## Herstellungsbeispiele

### Beispiel 1

$(CH_3)_3C-CH_2-N(CO-O-C_6H_5)_2$ (1)

Ein 4-Liter-Dreihalskolben wird mit Rührer und Rückflusskühler ausgerüstet, auf dem über einen Reitmeir-Aufsatz eine absteigende Destillationsbrücke angeschlossen wird. In den Kolben taucht möglichst tief eine grobporige Gaseinleitungsfritte. Auf dieser wird über eine Schliff-Verbindung ein Tropfrichter mit Druckausgleich angebracht. Das Gaszuführungsrohr der Fritte verbindet man mit der Stickstoffleitung sowie mit einer Quecksilber-Tauchung. Der Rückflusskühler wird bei 90–100°C betrieben, der absteigende Kühler bei $<$20°C. Alle Schliff-Verbindungen vor der Fritte sind gegen Überdruck zu sichern.

Man legt 2,54 ltr. = 3,13 kg (20 Mol) Kohlensäurephenylester-chlorid vor, erhitzt zum Rückfluss und tropft in die Gaseinleitungsfritte unter starkem Rühren innerhalb 8–9 Stunden gleichmässig eine Lösung von 174,0 g (2 Mol) Neopentylamin in 500 ml Petrolether. Dabei wird zusätzlich ein kräftiger Stickstoffstrom durchgeleitet. Die Innentemperatur soll 185°C nicht unterschreiten; gegen Ende der Reaktion werden 189–190°C erreicht. Wegen geringer Verkrustung der Fritte stellt sich während der Anfangsphase (5–30 Min. nach Zutropfbeginn) in der Fritte ein Überdruck bis zu 60 mm Hg (80 mbar) ein, der schliesslich auf 10–15 mm Hg (ca. 13–20 mbar) abfällt. Der verwendete Petrolether wird im absteigenden Kühler zusammen mit etwas Neopentyl-isocyanat und wenig Kohlensäure-phenylester-chlorid kondensiert.

Nach Ende der Eintropfzeit kocht man noch eine Stunde unter Rückfluss und weiterem Durchleiten von Stickstoff.

Eine gaschromatographische Analyse dieser Reaktionslösung liefert – ohne Berücksichtigung des im Überschuss eingesetzten Kohlensäure-phenylester-chlorids – folgende Werte:

14,3% – Diphenylcarbonat
83,9% – N-Neopentyl-imido-dicarbonsäure-diphenylester (1)
0,7% – Orthokohlensäure-tetraphenylester.

Die erhaltene Reaktionslösung wird destilliert aufgearbeitet:

Nach Abtrennen von wenig Vorlauf destilliert man die Hauptmenge des überschüssigen Kohlensäure-phenylester-chlorids (Siedepunkt: 76–78°C/19 mbar) ab, bis eine Sumpftemperatur von 140°C erreicht ist. Auf diese Weise gewinnt man 1,94 ltr. (=2,39 kg) eines 99,5%igen Esterchlorids zurück, das für die gleiche Umsetzung wiederverwendet werden kann.

Anschliessend destilliert man im Hochvakuum über eine kurze Vigreux-Kolonne Restmengen des Kohlensäure-phenylester-chlorids sowie das Diphenylcarbonat ab, bis eine Siedetemperatur von 160°C bei 0,2 mbar erreicht ist. Man erhält 578 g eines erstarrenden Rückstandes mit einer gaschromatographisch bestimmten Reinheit von 98,5%, entsprechend einer Ausbeute von 87% der Theorie an N-Neopentyl-imido-dicarbonsäure-diphenylester (1); Schmelzpunkt 81°C (aus Petrolether); Siedepunkt 156°C/0,02 mbar.

In analoger Weise werden die in der folgenden Tabelle aufgeführten Verbindungen hergestellt:

Tabelle

$R^1-N(CO-OR^2)_2$ (I)

| Beispiel Nr. | $R^1$ | $R^2$ | Schmelzpunkt (°C) | Siedepunkt (°C/mbar) |
|---|---|---|---|---|
| 2 | $CH_3-$ | $-C_6H_5$ | 102–105 | |
| 3* | $(CH_3)_2CH-$ | $-C_6H_5$ | 35– 37 | 155/0,07 |
| 4* | $C_2H_5-\underset{CH_3}{\underset{\vert}{CH}}-$ | $-C_6H_5$ | | 165–170/0,2 |

Tabelle (Fortsetzung)

$R^1-N(CO-OR^2)_2$ (I)

| Beispiel Nr. | $R^1$ | $R^2$ | Schmelzpunkt (°C) | Siedepunkt (°C/mbar) |
|---|---|---|---|---|
| 5* | $(CH_3)_2CH-CH_2-$ | $-C_6H_5$ | 40 | 160/0,1 |
| 6* | $(CH_3)_3C-$ | $-C_6H_5$ | 132 | 150/0,1 |
| 7 | $(CH_3)_3C-CH_2-$ | 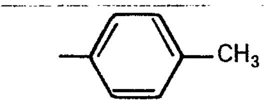 | 71- 73 | 172/0,008 |
| 8 | $(CH_3)_3C-CH_2-$ | 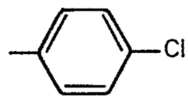 | 72- 73 | 182/0,03 |
| 9* | $(CH_3)_3C-CH_2-$ | 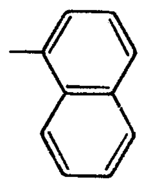 | 92- 93 | |
| 10 | $CH_3$<br>\|<br>$C_2H_5-C-$<br>\|<br>$CH_3$ | $-C_6H_5$ | 95- 97 | 150/0,05 |
| 11 | $(CH_3)_3C-CH-$<br>\|<br>$CH_3$ | $-C_6H_5$ | 40- 42 | 159/0,15 |
| 12 | $CH_3$<br>\|<br>$C_2H_5-C-$<br>\|<br>$CH_3$ | $-C_6H_5$ | 49- 50 | 178/0,05 |
| 13 | $[(CH_3)_2CH-]_2CH-$ | $-C_6H_5$ | | 158-161/0,02 |
| 14 | $C_4H_9-CH-CH_2$<br>\|<br>$C_2H_5$ | $-C_6H_5$ | | 182-185/0,06 |
| 15 | $C_7H_{15}-CH-$<br>\|<br>$CH_3$ | $-C_6H_5$ | | 173/0,09 |
| 16* | $(C_2H_5)_2CH-$ | $-C_6H_5$ | 54- 56 | 140/0,12 |
| 17 | $CH_2=CH-CH_2-$ | $-C_6H_5$ | | 153/0,04 |
| 18* | 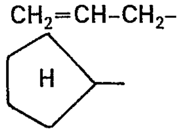 | $-C_6H_5$ | 53 | 160/0,1 |
| 19* | 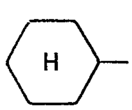 | $-C_6H_5$ | 85 | 175/0,09 |
| 20 | $(CH_3)_3C-$ 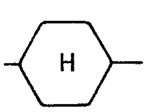 | $-C_6H_5$ | | 186-200/0,001 |

Tabelle (Fortsetzung)

$R^1-N(CO-OR^2)_2$ (I)

| Beispiel Nr. | $R^1$ | $R^2$ | Schmelzpunkt (°C) | Siedepunkt (°C/mbar) |
|---|---|---|---|---|
| 21 | 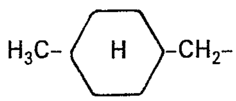 | $-C_6H_5$ | | 185–188/0,06 |
| 22 | 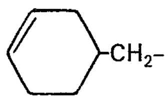 | $-C_6H_5$ | | 195–200/0,09 |
| 23 | 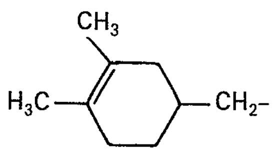 | $-C_6H_5$ | | 200–205/0,07 |
| 24 | 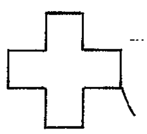 | $-C_6H_5$ | 57– 62 | 210/0,6 |
| 25 | 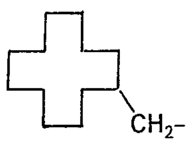 | $-C_6H_5$ | | 240–250/0,1 |
| 26 | 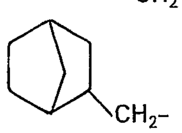 | $-C_6H_5$ | 84– 86 | 202/0,02 |
| 27 | 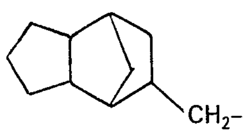 | $-C_6H_5$ | 92– 94 | |
| 28 | 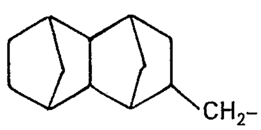 | $-C_6H_5$ | | 232–240/0,07 |
| 29 | 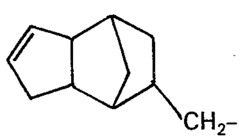 | $-C_6H_5$ | | 215–220/0,07 |
| 30 | $Cl(CH_2)_2-$ | $-C_6H_5$ | | 177–190/0,1 |
| 31* | $F_3C-CH_2-$ | $-C_6H_5$ | 76 | 140/0.3 |
| 32 | $Cl_2FC-(CH_2)_2-$ | $-C_6H_5$ | 68– 70 | 187/0,008 |
| 33 | $F_3C-(CH_2)_2-$ | $-C_6H_5$ | 66– 69 | 147–153/0,3 |
| 34 | $CH_3-CF_2-CH_2-$ | $-C_6H_5$ | 60– 62 | 160–164/0,08 |
| 35 | $F_3C-CH(CH_3)-$ | $-C_6H_5$ | | 150/0,17 |

Tabelle (Fortsetzung)

$R^1$-N(CO-O$R^2$)$_2$ (I)

| Beispiel Nr. | $R^1$ | $R^2$ | Schmelzpunkt (°C) | Siedepunkt (°C/mbar) |
|---|---|---|---|---|
| 36 | Cl-$(CH_2)_6$- | -$C_6H_5$ | | 210/0,1 |
| 37 | $H_3C$-O-$(CH_2)_2$- | -$C_6H_5$ | | 168/0,1 |
| 38 | $H_3C$-O-$(CH_2)_3$- | -$C_6H_5$ | | 168–170/0,08 |
| 39 | NC-$(CH_2)_5$- | -$C_6H_5$ | | 230–235/0,1 |
| 40 | $C_2H_5$-O-CO-$(CH_2)_2$- | -$C_6H_5$ | | 190–193/0,08 |
| 41 | $C_6H_5$- | -$C_6H_5$ | 124–125 | |
| 42 | 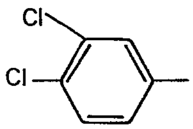 | -$C_6H_5$ | 97–100 | |
| 43* | 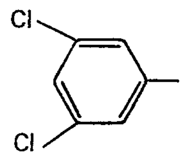 | -$C_6H_5$ | 178–180 | |
| 44 | $C_6H_5$-$CH_2$- | -$C_6H_5$ | 70– 72 | 191/0,01 |
| 45 | 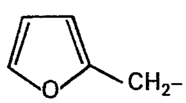 | -$C_6H_5$ | | 190–200/0,1 |
| 46 | 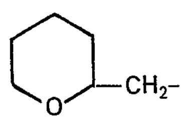 | -$C_6H_5$ | 78– 79 | 176/0,007 |

Die mit * gekennzeichneten Verbindungen sind erstmals in der vorgängigen Patentanmeldung gemäss EP-A-O 034 750 beschrieben worden, die übrigen sind neu.

**Patentansprüche**

1. Verfahren zur Herstellung von N-subtituierten Imidodicarbonsäure-diarylestern der allgemeinen Formel I

$R^1$-N(CO-O$R^2$)$_2$ (I),

in welcher
$R^1$ für einen geradkettigen oder verzweigten Alkylrest mit 1–10 C-Atomen, der gegebenenfalls durch Niederalkoxy, Niederalkylmercapto, Halogen, Cyano oder Nitro substituiert sein kann; einen Alkenylrest mit 3-8 C-Atomen; einen Alkinylrest mit 3-8 C-Atomen; einen cycloaliphatischen Rest mit 3-8 C-Atomen, der gegebenenfalls durch Niederalkyl substituiert sein kann; einen araliphatischen Rest mit 7-12 C-Atomen, wobei das aromatische Ringsystem gegebenenfalls durch Halogen, Nitro, Trifluormethyl, Cyano, Niederalkyl und/oder Niederalkoxy substituiert sein kann; einen aromatischen Rest mit 6-12 C-Atomen, der gegebenenfalls durch Halogen, Nitro, Trifluormethyl, Cyano, Niederalkyl und/ oder Niederalkoxy substituiert sein kann, oder für einen heterocyclischen Rest mit 5-6 Ringatomen, wobei 1-3 Heteroatome im Ringsystem vorhanden sein können, steht, und
$R^2$ für einen gegebenenfalls durch Chlor, Methyl und/oder Methoxy substituierten Phenyl- oder Naphthylrest steht,
dadurch gekennzeichnet, dass man primäre Amine der allgemeinen Formel II

$R^1$-$NH_2$ (II),

in welcher $R^1$ die oben angegebene Bedeutung hat,

mit Kohlensäure-arylester-halogeniden der allgemeinen Formel III

X-CO-OR$^2$ (III),

in welcher
R$^2$ die oben angegebene Bedeutung hat und
X für Halogen steht,
in Gegenwart eines Verdünnungsmittels, ohne Zusatz eines Säurebindemittels, bei Temperaturen zwischen 100 und 300°C umsetzt.

2. Verfahren nach Anspruch 1, dadurch gekennzeichnet, dass man die Umsetzung bei Temperaturen zwischen 170°C und 250°C durchführt.

3. Verfahren nach Anspruch 1, dadurch gekennzeichnet, dass man das Kohlensäure-arylester-halogenid (III) in überstöchiometrischen Mengen einsetzt.

4. Verfahren nach Anspruch 1, dadurch gekennzeichnet, dass man das Kohlensäure-arylester-halogenid (III) vorlegt und bei der gewünschten Reaktionstemperatur eine Lösung des primären Amins (II) in einem Verdünnungsmittel zuführt, wobei als Verdünnungsmittel ein Kohlenwasserstoff, vorzugsweise Petrolether, oder ein chlorierter Kohlenwasserstoff verwendet wird.

5. Verfahren nach Anspruch 1, dadurch gekennzeichnet, dass man Kohlensäure-arylester-halogenide der Formel (III) einsetzt, in welcher
R$^2$ die oben angegebene Bedeutung hat und
X für Chlor oder Fluor steht.

6. Verfahren nach Anspruch 1, dadurch gekennzeichnet, dass man Neopentylamin mit Kohlensäure-phenylester-chlorid umsetzt.

**Claims**

1. Process for the preparation of N-substituted imidodicarboxylic acid diarylesters of the general formula I

$R^1$-N(CO-OR$^2$)$_2$ (I)

in which
R$^1$ represents a straight-chain or branched alkyl radical which has 1-10 C atoms and can optionally be substituted by lower alkoxy, lower alkylmercapto, halogen, cyano or nitro ; an alkenyl radical with 3-8 C atoms; an alkinyl radical with 3-8 C atoms; a cycloaliphatic radical which has 3-8 C atoms and can optionally be substituted by lower alkyl; an araliphatic radical with 7-12 C atoms, it being possible for the aromatic ring system optionally to be substituted by halogen, nitro, trifluoromethyl, cyano, lower alkyl and/or lower alkoxy; an aromatic radical with 6-12 C atoms, which can optionally be substituted by halogen, nitro, trifluoromethyl, cyano, lower alkyl and/or lower alkoxy, or a heterocyclic radical with 5-6 ring atoms, it being possible for 1-3 hetero atoms to be present in the ring system, and
R$^2$ represents a phenyl or naphtyl radical which is optionally substituted by chlorine, methyl and/or methoxy,
characterised in that primary amines of the general formula II

$R^1$-$NH_2$ (II)

in which
R$^1$ has the abovementioned meaning,
are reacted with carbonic acid aryl ester halides of the general formula III

X-CO-OR$^2$ (III)

in which
R$^2$ has the abovementioned meaning and
X represents halogen,
in the presence of a diluent, without the addition of an acid-binding agent, at temperatures between 100 and 300°C.

2. Process according to Claim 1, characterised in that the reaction is carried out at temperatures between 170°C and 250°C.

3. Process according to Claim 1, characterised in that the carbonic acid aryl ester halide (III) is employed in amounts greater than the stoichiometric amount.

4. Process according to Claim 1, characterised in that the carbonic acid aryl ester halide (III) is initially introduced and a solution of the primary amine (II) in a diluent is added at the desired reaction temperature, a hydrocarbon, preferably petroleum ether, or a chlorinated hydrocarbon being used as the diluent.

5. Process according to Claim 1, characterised in that carbonic acid aryl ester halides of the formula (III) are used, in which

R$^2$ has the abovementioned meaning and
X represents chlorine or fluorine.

6. Process according to Claim 1, characterised in that neopentylamine is reacted with carbonic acid phenyl ester chloride.

**Revendications**

1. Procédé de préparation d'esters diaryliques d'acides imidodicarboxyliques substitués à l'azote, répondant à la formule générale I

$R^1$-N(CO-OR$^2$)$_2$ (I)

dans laquelle R$^1$ représente un groupe alkyle à chaîne droite ou ramifiée en $C_1$–$C_{10}$ portant éventuellement des substituants alcoxy inférieur, alkylmercapto inférieur, halogéno, cyano ou nitro; un reste alcényle en $C_3$–$C_8$; un reste alcynyle en $C_3$–$C_8$; un reste cycloaliphatique en $C_3$–$C_8$ éventuellement substitué par des groupes alkyle inférieurs; un reste araliphatique en $C_7$–$C_{12}$, le système cyclique aromatique pouvant porter éventuellement des substituants halogéno, nitro, trifluorométhyle, cyano, alkyle inférieur et/ou alcoxy inférieur; un reste aromatique en $C_6$–$C_{12}$ portant éventuellement des substituants halogéno, nitro, trifluorométhyle, cyano, alkyle inférieur et/ou alcoxy inférieur, ou un reste hétérocyclique contenant 5 à 6 atomes cycliques, le système cyclique pouvant contenir 1 à 3 hétéroatomes, et
$R^2$ représente un reste phényle ou naphtyle portant éventuellement des substituants chloro, méthyle et/ou méthoxy,
caractérisé en ce que l'on fait réagir des amines primaires de formule générale II

$R^1$-$NH_2$ (II)

dans laquelle $R^1$ a la signification indiquée ci-dessus,
avec des halogénures-esters aryliques de l'acide carbonique répondant à la formule générale III

X-CO-$OR^2$ (III)

dans laquelle
$R^2$ a la signification indiquée ci-dessus et
X représente un halogène,
en présence d'un diluant, sans adjonction d'un agent fixant les acides, à des températures de 100 à 300°C.

2. Procédé selon la revendication 1, caractérisé en ce que la réaction est effectuée à des températures de 170 à 250°C.

3. Procédé selon la revendication 1, caractérisé en ce que l'on utilise l'halogénure-ester arylique de l'acide carbonique (III) en quantité supérieure à la quantité stœchiométrique.

4. Procédé selon la revendication 1, caractérisé en ce que l'on introduit d'abord dans l'appareil l'halogénure-ester arylique de l'acide carbonique (III) et on ajoute, à la température de réaction voulue, une solution de l'amine primaire (II) dans un diluant, le diluant utilisé consistant en un hydrocarbure, de préférence l'éther de pétrole, ou un hydrocarbure chloré.

5. Procédé selon la revendication 1, caractérisé en ce que l'on utilise des halogénures-esters aryliques de l'acide carbonique de formule (III) dans laquelle
$R^2$ a la signification indiquée ci-dessus et
X représente le chlore ou le fluor.

6. Procédé selon la revendication 1, caractérisé en ce que l'on fait réagir la néopentylamine avec le chlorure-ester phénylique de l'acide carbonique.